Europäisches Patentamt

European Patent Office

Office européen des brevets

(19)

(11) Veröffentlichungsnummer : **0 133 668**
**B1**

(12) EUROPÄISCHE PATENTSCHRIFT

(45) Veröffentlichungstag der Patentschrift :
13.04.88

(51) Int. Cl.⁴ : **C 07 C175/00**

(21) Anmeldenummer : **84108851.1**

(22) Anmeldetag : **26.07.84**

(54) Verfahren zur Herstellung von Jononen.

(30) Priorität : **06.08.83 DE 3328440**

(43) Veröffentlichungstag der Anmeldung :
**06.03.85 Patentblatt 85/10**

(45) Bekanntmachung des Hinweises auf die Patenterteilung : **13.04.88 Patentblatt 88/15**

(84) Benannte Vertragsstaaten :
**CH DE FR GB LI NL**

(56) Entgegenhaltungen :
**DE-A- 1 568 108**
**DE-A- 1 917 132**
**FR-A- 1 550 943**
**US-A- 2 877 271**

(73) Patentinhaber : **BASF Aktiengesellschaft**
**Carl-Bosch-Strasse 38**
**D-6700 Ludwigshafen (DE)**

(72) Erfinder : **Hertel, Otto, Dr.**
**Erzbergerstrasse 34**
**D-6700 Ludwigshafen (DE)**
Erfinder : **Kiefer, Hans, Dr.**
**Im Sandgarten 5**
**D-6706 Wachenheim (DE)**
Erfinder : **Arnold, Lothar, Dr.**
**Hausackerweg 11**
**D-6900 Heidelberg (DE)**

## Beschreibung

Die Erfindung betrifft ein verbessertes Verfahren zur kontinuierlichen Herstellung von α- und/oder β-Jonon durch Cyclisieren von Pseudojonon mittels konzentrierter Schwefelsäure in Gegenwart von organischen Lösungs- oder Verdünnungsmitteln und durch Verdünnen des Reaktionsgemisches mit Wasser.

Es ist bekannt, daß bei der Cyclisierung von Pseudojonon in Gegenwart von Säuren wie Schwefelsäure oder Phosphorsäure ein Gemisch von α- und β-Jonon erhalten wird. Das Verhältnis der Mengen, in welchen diese Verbindungen entstehen, ist stark von den Bedingungen, unter die Reaktion stattfindet, abhängig.

Da sowohl α-Jonon als auch β-Jonon von großer technischer Bedeutung sind, hat es nicht an Versuchen gefehlt, ein möglichst vorteilhaftes Verfahren zu deren Herstellung zu entwickeln.

Besonders bewährt haben sich Verfahren zur Cyclisierung von Pseudojonon mit konzentrierter Schwefelsäure. Da diese Umsetzung stark exotherm verläuft, ist es sehr wichtig, die entstehende Reaktionswärme möglichst schnell abzuführen, um örtliche Überhitzungen zu vermeiden. Zu diesem Zweck wurden dem Reaktionsgemisch gemäß den bekannten Verfahren Verdünnungsmittel zugegeben. So werden beispielsweise, gemäß DE-C- 10 80 105 und DE-A- 16 68 505 aliphatische oder cycloaliphatische Kohlenwasserstoffe verwendet. Nachteilig an diesem Verfahren ist, daß sich in den Reaktionsgefäßen relativ schnell Harze abscheiden, die den kontinuierlichen Betrieb stören.

Gemäß der IN-PS 77 225 wird die Umsetzung in Gegenwart von aliphatischen Chlorkohlenwasserstoffen wie Methylenchlorid, Ethylendichlorid, Chloroform und Tetrachlorkohlenstoff bei Temperaturen von — 10 °C bis + 10 °C durchgeführt.

Gemäß der Beschreibung der DE-A- 15 68 108 ist dieses indische Verfahren nachteilig, da die aliphatischen Chlorkohlenwasserstoffe mit Schwefelsäure Chlorwasserstoff abspalten, wodurch die verwendeten Apparate in kurzer Zeit korrodieren. Zur Vermeidung dieser Nachteile wird empfohlen, die Cyclisierung bei — 25 bis + 10 °C in einem Gemisch aus niedrig-siedenden Kohlenwasserstoffen und Chlorkohlenwasserstoffen durchzuführen. Nachteilig an beiden letztgenannten Verfahren ist, daß man die Reaktionstemperatur mit aufwendigen Kühlmitteln niedrig halten muß, um gute Jonon-Ausbeuten zu erzielen.

Weiterhin sind Verfahren bekannt, bei denen die beträchtliche Cyclisierungswärme durch Siedekühlung mit Flüssiggasen abgeführt wird. So arbeitet man gemäß dem Verfahren der DE-A- 16 68 496 mit flüssigem Schwefeldioxid, dem Verfahren der DE-A- 16 68 505 (entspricht FR-A- 1550943) mit Propan, Butan oder Isobutan und dem Verfahren der DE-A- 19 17 132 mit Methylchlorid und bei Temperaturen von — 25 °C bis Raumtemperatur, vorzugsweise Temperaturen unterhalb von + 10 °C.

Die gemäß diesen Verfahren erzielten Ergebnisse sind im allgemeinen recht gut. Nachteilig hieran ist der große Aufwand, der nötig ist, um das bei der Reaktion verdampfte Gas wieder zu verflüssigen.

Aus der CS-A- 179 046, der SU-A- 458 540 und der SU-A- 547 445 sind ferner Verfahren zur Herstellung von β-Jonon bekannt, bei denen eine gute Durchmischung der Reaktanten und eine schnelle Wärmeabfuhr dadurch erreicht werden, daß man einen Dünnfilmreaktor verwendet. Nachteilig an den beiden letztgenannten Verfahren ist, daß man pro m² Dünnfilmfläche und Stunde nur etwa 3 bis 6 kg β-Jonon erhält, und daß somit eine Übertragung in den industriellen Maßstab zu riesigen Apparaturen führen würde. Nachteilig an dem Verfahren des tschechischen Patents ist, daß man zur Erzielung guter Ausbeuten bei Temperaturen zwischen 10 und 15 °C arbeiten muß wodurch wiederum teure Kühlmittel notwendig werden.

Bei allen bekannten Verfahren bildet sich immer ein Gemisch von α- und β-Jonon. Nach den DE-A- 10 80 105, DE-A- 16 68 496 und DE-A- 16 68 505 erhält man bei Reaktionstemperaturen von —20 bis 0 °C bevorzugt β-Jonon, während bei Temperaturen von —10 bis 25 °C der α-Jonon-Gehalt ansteigt. β-Jonon ist ein wichtiges Vorprodukt für die technische Herstellung von Vitamin A. Ein hoher Gehalt an α-Jonon, führt dabei zu einer Verminderung der Ausbeute. Reines α-Jonon dagegen ist ein begehrter Riechstoff, in welchem ein höherer Gehalt an β-Jonon störend wirken würde.

Es war daher die Aufgabe der Erfindung, ein Verfahren zu entwickeln, mit dem sowohl möglichst reines α-Jonon als auch möglichst reines β-Jonon auf möglichst vorteilhafte Weise in hohen Ausbeuten und Raum-Zeit-Ausbeuten hergestellt werden können.

Es wurde nun ein sehr vorteilhaftes Verfahren zur kontinuierlichen Herstellung von Jononen durch Cyclisieren von Pseudojonon mittels konzentrierter Schwefelsäure in Gegenwart von organischen Lösungs- oder Verdünnungsmitteln unter Kühlung und durch Verdünnen des Reaktionsgemisches mit Wasser gefunden, das dadurch gekennzeichnet, ist, daß man das Pseudojonon mit einem unter den Reaktionsbedingungen zwischen 25 und 65 °C siedenden aliphatischen oder cycloaliphatischen Kohlenwasserstoff oder einem aliphatischen Chlorkohlenwasserstoff und der Schwefelsäure unter intensiver Durchmischung und Siedekühlung durch teilweises oder vollständiges Verdampfen des im Reaktionsgemisch enthaltenen Lösungsmittels so zusammenführt, daß die Temperatur des Reaktionsgemisches zwischen 30 und 55 °C, insbesondere zwischen 35 und 45 °C liegt und die Verweilzeit des Reaktionsgemisches bis zum Verdünnen mit Wasser oder verdünnter Schwefelsäure 0,05 bis 20, vorzugsweise 0,1 bis 5 Sekunden beträgt.

Besonders vorteilhaft gestaltet sich das erfindungsgemäße Verfahren, wenn man das Pseudojonon mit einem unter Normaldruck zwischen 25 und 65 °C siedenden Lösungsmittel, vorzugsweise Methylenchlorid oder Chloroform, insbesondere Methylenchlorid, und der Schwefelsäure unter intensiver Durchmischung und Siedekühlung durch teilweises Verdampfen des im Reaktionsgemisch enthaltenden Lösungsmittels zusammenführt.

Einen weiteren Vorteil bringt das erfindungsgemäße Verfahren, wenn man auch das Verdünnen des Reaktionsgemisches mit Wasser unter Siedekühlung durch Verdampfen des im Reaktionsgemisch enthaltenen Lösungsmittels vornimmt.

Es wurde weiterhin gefunden, daß das Verhältnis von α-Jonon zu β-Jonon im Reaktionsprodukt sehr stark von der angewandten Schwefelsäuremenge beeinflußt wird.

Überwiegend α-Jonon erhält man bei dem erfindungsgemäßen Verfahren, wenn man etwa 2 bis 3 mol konzentrierte Schwefelsäure pro Mol Pseudojonon verwendet, überwiegend β-Jonon, wenn man mehr als 5 mol, also etwa 5 bis 15 mol Schwefelsäure pro Mol Pseudojonon verwendet.

Die gefundenen Ergebnisse sind überraschend, da bei nahezu allen bekannten Verfahren hervorgehoben wurde, daß bei der Cyclisierung von Pseudojonon mit Schwefelsäure nur gute Ausbeuten an Jononen, insbesondere an β-Jonon, erhalten werden, wenn man bei möglichst tiefen Temperaturen arbeitet.

Die erfindungsgemäße Umsetzung erfolgt in so guten Raum-Zeit-Ausbeuten, daß man mit recht kleinen Reaktionsgefäßen auskommt, wodurch die Verwendung von Glasgeräten möglich wird, so daß Korrosionsprobleme, die bei Verwendung von aliphatischen Halogenkohlenwasserstoffen als Lösungsmittel auftreten könnten, vermieden werden.

Die erfindungsgemäße Cyclisierungsreaktion kann prinzipiell in geeigneten Dünnschichtreaktoren, in einer einfachen Düse, in einem statischen Mischer oder in einer beliebigen Mischstrecke durchgeführt werden.

Besonders vorteilhaft läßt sich die Cyclisierung in einer kurzen Mischstrecke durchführen.

Die destillative Rückgewinnung des verdampften Lösungsmittels gestaltet sich relativ einfach, da zur Kondensation die Verwendung von Flußwasser als Kühlmittel ausreichend ist.

Die Konzentration der bei der Cyclisierung verwendeten Schwefelsäure kann zwischen 90 und 100 Gew.% liegen. Vorzugsweise verwendet man handelsübliche 95 bis 98 %ige Schwefelsäure. Pro Mol Pseudojonon verwendet man im allgemeinen 2 bis 15 mol, vorzugsweise 2 bis 7 mol Schwefelsäure. Bei Verwendung von 2 bis 3 mol Schwefelsäure pro Mol Pseudojonon erhält man überwiegend α-Jonon, während man beim Einsatz von mehr als 5 mol Schwefelsäure pro Mol Pseudojonon β-Jonon mit einem Gehalt von weniger als 2 % α-Jonon erhält.

Als Lösungsmittel kommen im wesentlichen aliphatische oder cycloaliphatische Kohlenwasserstoffe sowie aliphatische Chlorkohlenwasserstoffe, die unter den Reaktionsbedingungen bei Temperaturen zwischen 25 und 65 °C sieden, in Betracht. Genannt seien beispielsweise Pentan, Hexan, Isopentant, Cyclohexan, Methylenchlorid, Chloroform, Tetrachlorkohlenstoff, 1,2-Dichlorethan sowie 1,1,1-Trichlorethan. Bei Lösungsmitteln, die unter Normalbedingungen oberhalb von 65 °C sieden, werden die optimalen Reaktionstemperaturen von etwa 35 bis 45 °C durch Vermindern des Druckes im Reaktionsgefäß eingestellt. Von besonderem Vorteil ist es natürlich, solche Lösungsmittel zu verwenden, die unter Normalbedingungen bei Temperaturen zwischen 25 und 65 °C sieden. Vorzugsweise verwendet man Chloroform (Kp = 61,3 °C) oder insbesondere Methylenchlorid (Kp = 41,6 °C).

Die Lösungsmittelmenge ist in weiten Grenzen variierbar. Die besten Ergebnisse erzielt man jedoch, wenn man pro Volumenteil Pseudojonon etwa 1 bis 5, vorzugsweise 1,5 bis 3 Volumenteile des Lösungsmittels verwendet.

Die Lösungsmittelmenge hängt natürlich davon ab, ob auch die beim Verdünnen des Reaktionsgemisches mit Wasser auftretende Reaktionswärme durch Siedekühlung abgeführt werden soll, was besonders vorteilhaft ist.

Nach beendeter Reaktion wird das Reaktionsgemisch unmittelbar mit Wasser verdünnt, und zwar verwendet man in der Regel 0,8 bis 21 Wasser pro kg Schwefelsäure. Hierbei empfiehlt es sich dafür zu sorgen, daß die Temperatur infolge der Hydratisierungswärme der Schwefelsäure nicht über 50 °C ansteigt. Mann kann dies bewirken, indem man die Wärme ebenfalls durch Siedekühlung mittels entsprechender Mengen des Lösungsmittels abführt.

Die bei dem Verfahren als Nebenprodukt anfallende verdünnte Schwefelsäure läßt sich umso besser verwerten, je höher deren Konzentration ist. Es ist daher technisch und wirtschaftlich zweckmäßig, die Verdünnung des Reaktionsgemisches aus dem Lösungsmittel, den Jononen und der konzentrierten Schwefelsäure nicht mit reinem Wasser, sondern mit verdünnter, etwa 20 bis 50 gew.%iger Schwefelsäure vorzunehmen, wobei es sich naturgemäß anbietet, diejenige verdünnte Säure nach Zwischenkühlung zu verwenden, die bei dem Verfahren entsteht. Beträgt die Konzentration dieser Säure vor der Verdünnung des Reaktionsgemisches z. B. 45 Gew.%, so erhält man wegen der neu zugeführten konzentrierten Schwefelsäure nach der Verdünnung eine höher konzentrierte, beispielsweise 60 gew.%ige Säure, von der im stationären Betrieb ein Teil aus dem System abgeführt wird, während der restliche Teil gekühlt, mit frischem Wasser wieder auf 45 Gew.% gestellt und recyclisiert wird.

Mit Hilfe des erfindungsgemäßen Verfahrens können α- und β-Jonon auf technisch einfache und vorteilhafte Weise in sehr guten Ausbeuten hergestellt werden.

## Beispiel 1

### a) Beschreibung der Apparatur

Die Mischkammer bestand aus einem mit Raschigringen gefüllten, senkrecht angeordneten Glasrohr von 2 cm Innendurchmesser und 30 cm Höhe, an dessen oberen Ende sich die getrennten Zuleitungen für die Pseudojononlösung und die konzentrierte Schwefelsäure befanden.

Unterhalb des Rohres war ein Zwischenstück von 5 cm Höbe angeordnet, welches mit einer Düse zum Einspritzen des Verdünnungswassers bzw. der verdünnten Schwefelsäure versehen war.

Das Zwischenstück war seinerseits auf einem Kühler angeordnet, welcher zur Kondensation des Lösungsmittels diente. Unterhalb des Kühlers befand sich ein Abscheider zur Phasentrennung mit den entsprechenden Entnahmeleitungen.

### b) Reaktionsdurchführung

In die oben beschriebene Apparatur wurden innerhalb von 4 Minuten über 2 Glasrohre gleichzeitig eine Lösung von 600 g Pseudojonon im 1.200 ml Methylenchlorid und 1.130 ml konzentrierte Schwefelsäure (96 %ig.) eingeführt. Die beim Zusammentreffen der beiden Lösungen freiwerdende Reaktionswärme wurde zum großen Teil durch das Verdampfen von Methylenchlorid abgeführt, so daß das Reaktionsgemisch etwa eine Temperatur von 41 °C aufwies. Durch den gebildeten Methylenchloriddampf wurde das Reaktionsgemisch sehr schnell durch die Mischkammer getrieben, so daß sich Verweilzeiten von nur etwa 0,5 Sekunden ergaben.

Das die Mischkammer verlassende, etwa 41 °C warme Reaktionsgemisch wurde sofort durch die Düse mit insgesamt 2.000 ml (500 ml/Minute) Wasser versetzt, wonach das Gemisch den Kühler passierte und in den Abscheider gelangte. Hier wurde die Methylenchloridphase von der wäßrig-schwefelsauren Phase getrennt. Die wäßrige Phase wurde noch zweimal mit je 400 ml Methylenchlorid extrahiert. Die vereinigten Extrakte wurden mit 15 %iger Sodalösung auf pH 8 bis 9 eingestellt. Anschließend wurde das Lösungsmittel destillativ abgetrennt und der Rückstand bei 0,1 mbar destilliert. Die Ausbeute betrug 82,7 % β-Jonon und 2,7 % α-Jonon. Der Rückstand bestand aus einem gut fließfähigen Harz, das umweltfreundlich verbrannt werden kann.

## Beispiele 2 bis 8

Analog Beispiel 1 b wurden in die in Beispiel 1a beschriebene Apparatur innerhalb der aus der Tabelle ersichtlichen Zeit gleichzeitig eine Lösung von jeweils 600 g Pseudojonon in der aus der Tabelle ersichtlichen Menge des dort angegebenen Lösungsmittels und die ebenfalls aus der Tabelle ersichtliche Menge konzentrierter Schwefelsäure eingeführt. Temperatur im Mischer sowie die mittlere Verweilzeit sind in der Tabelle angegeben. Das die Mischkammer verlassende Reaktionsgemisch wurde sofort über die Düse mit insgesamt der aus der Tabelle ersichtlichen Menge Wasser versetzt, und lief dann über den Kühler in den Abscheider. Die Aufarbeitung des Reaktionsgemisches erfolgte analog Beispiel 1 b. Die erzielten Ausbeuten an α- und β-Jonon sind in der Tabelle angegeben.

In Beispiel 8 wurde das die Mischkammer verlassende Reaktionsgemisch nicht mit Wasser, sondern mit einer 40 gew. %igen Schwefelsäure versetzt.

(Siehe Tabelle 1 Seite 5 f.)

4

Tabelle 1

| Bei-spiel | Lösungs-mittel | Menge [ml] | konz. $H_2SO_4$ [ml] | Versuchs-dauer [Min.] | Temperatur im Mischer [°C] | mittlere Verweil-zeit [Sekunden] | Wasser [ml] | Ausbeute | |
|---|---|---|---|---|---|---|---|---|---|
| | | | | | | | | ß-Jonon | α-Jonon |
| 2 | $CH_2Cl_2$ | 1200 | 1150 | 2 | 41 | 0,5 | 2000 | 85 % | 3 % |
| 3 | $CH_2Cl_2$ | 1100 | 1050 | 1,5 | 41 | 0,4 | 2300 | 89,7 % | 1,7 % |
| 4 | $CH_2Cl_2$ | 900 | 380 | 1,5 | 41 | 1,0 | 2000 | 33 % | 51 % |
| 5 | $CH_2Cl_2$ | 900 | 1070 | 6 | 41 | 1,5 | 3000 | 87,4 % | 1,2 % |
| 6 | $CHCl_3$ | 1200 | 1180 | 1,5 | 61 | 0,5 | 2000 | 84,6 % | 2,5 % |
| 7* | Hexan | 1400 | 1100 | 1,5 | 45 | 0,5 | 2000 | 81 % | 2,0 % |
| 8** | $CH_2Cl_2$ | 1200 | 1130 | 1,5 | 51 | 0,5 | 2000** | 88,9 % | 2,0 % |

* Versuch 7 wurde bei 1.300 mbar durchgeführt.
** Bei Versuch 8 wurde das Reaktionsgemisch mit 40 gew.%iger Schwefelsäure verdünnt.

## Beispiel 9

### a) Beschreibung der Apparatur

Als Reaktionsgefäß diente ein Dünnschichtverdampfer, der aus einem senkrecht stehenden, unten offenen Glasrohr von 40 cm Länge und 2,2 cm Durchmesser, bestand, das mit einem Metallrührer, der 4 bewegliche Flügelpaare trug, versehen war. Die Länge der 4 Flügelpaare betrug 20 cm und die damit bestrichene Fläche 0,014 m². Die beiden Reaktionskomponenten wurden über zwei getrennte Anschlüsse in den oberen Reaktionsraum gebracht. Das bei der Reaktion verdampfende Lösungsmittel wurde kondensiert und in den Reaktor zurückgeführt. Das ablaufende Reaktionsgemisch wurde in einem zweiten Dünnschichtreaktor mit Wasser zersetzt. Auch hier wurde das verdampfende Lösungsmittel kondensiert und zurückgeführt.

### b) Reaktionsdurchführung

Durch einen der beiden Anschlüsse des Reaktors, wurden unter kräftigem Rühren (800 Umdrehungen/Minute) kontinuierlich innerhalb von 10 Minuten eine Lösung von 600 g eines 96 %igen Pseudojonons in 1.200 ml Methylenchlorid und durch den anderen Anschluß gleichzeitig 1.000 ml einer 96 %igen Schwefelsäure eingeführt. Das mit 42 °C ablaufende Produkt wurde im nachgeschalteten Dünnfilmreaktor mit 2.000 ml Wasser hydrolysiert, die organische Phase abgetrennt, mit 2 %iger Natriumcarbonatlösung und anschließend mit Wasser neutral gewaschen, unter vermindertem Druck vom Lösungsmittel befreit und danach bei 0,2 mbar destilliert. Man erhielt 495 g eines 97 %igen β-Jonons. Das entspricht einer Ausbeute an β-Jonon von 83,3 % der Theorie. Auch hier fiel als Rückstand ein gut fließfähiges Harz an.

## Beispiel 10

Man arbeitet genau wie in Beispiel 9 beschrieben, nur daß man die Lösung von 600 g eines 96 %igen Pseudojonons in 1.200 ml Methylenchlorid und die 1.000 ml Schwefelsäure gleichzeitig innerhalb von 5 Minuten anstelle von 10 Minuten in den Reaktor einführte. Bei der Destillation wurden 490 g eines 98 %igen β-Jonons erhalten. Das entspricht einer Ausbeute von β-Jonon von 83,4 % der Theorie.

## Beispiel 11 bis 18

Die Beispiele 11 bis 18 wurden analog Beispiel 9 durchgeführt. Die Reaktionsparameter im einzelnen sowie die Ausbeuteangaben sind in der folgenden Tabelle zusammengestellt.

(Siehe Tabelle 2 Seite 7 f.)

Tabelle 2

| Bei-spiel | Lösungs-mittel | Menge [ml] | konz. $H_2SO_4$ [ml] | Druck [mbar] | Versuchs-dauer [Min] | Temperatur im Mischer [°C] | mittlere Verweil-zeit [Sekunden] | Wasser [ml] | Ausbeute | |
|---|---|---|---|---|---|---|---|---|---|---|
| | | | | | | | | | ß-Jonon | ⍺-Jonon |
| 11 | $CHCl_3$ | 1200 | 1100 | 280 | 11,5 | 35 | 2 | 2000 | 85 | 2,0 |
| 12 | $CCl_4$ | 1200 | 1040 | 210 | 11 | 40 | 2 | 2000 | 78 | 2,5 |
| 13 | $ClCH_2-CH_2-Cl$ | 1200 | 900 | 210 | 10 | 45 | 2 | 2000 | 83 | 2,4 |
| 14 | $CH_3-CCl_3$ | 1200 | 1070 | 250 | 12 | 42 | 2 | 2000 | 82 | 2,0 |
| 15 | Hexan | 1200 | 1070 | 250 | 11 | 35 | 2 | 2000 | 80 | 2,7 |
| 16 | Cyclohexan | 1200 | 1120 | 250 | 11 | 45 | 2 | 2000 | 76 | 1,8 |
| 17 | Pentan | 1200 | 1015 | 1013 | 9,5 | 40 | 2 | 3000 | 76 | 2,5 |
| 18* | $CHCl_3$ | 1200 | 1080 | 1013 | 10 | 48 | 2 | 2000 | 83 | 2,1 |

* bei diesem Versuch wurde der Dünnfilmreaktor mit Wasser gekühlt.

**Patentansprüche**

1. Verfahren zur kontinuierlichen Herstellung von Jononen durch Cyclisieren von Pseudojonon mittels konzentrierter Schwefelsäure in Gegenwart von organischen Lösungs- oder Verdünnungsmitteln unter Kühlung und durch Verdünnen des Reaktionsgemisches mit Wasser, dadurch gekennzeichnet, daß man das Pseudojonon mit einem unter den Reaktionsbedingungen zwischen 25 und 65 °C siedenden aliphatischen oder cycloaliphatischen Kohlenwasserstoff oder einem aliphatischen Chlorkohlenwasserstoff und der Schwefelsäure unter intensiver Durchmischung und Siedekühlung durch teilweises oder vollständiges Verdampfen des im Reaktionsgemisch enthaltenen Lösungsmittels so zusammenführt, daß die Temperatur des Reaktionsgemisches zwischen 30 und 55 °C liegt und die Verweilzeit des Reaktionsgemisches bis zum Verdünnen mit Wasser 0,05 bis 20, vorzugsweise 0,1 bis 5 Sekunden beträgt.

2. Verfahren nach Anspruch 1, dadurch gekennzeichnet, daß man das Pseudojonon mit einem unter Normaldruck zwischen 30 und 55 °C siedenden Lösungsmittel und der Schwefelsäure unter intensiver Durchmischung und Siedekühlung durch teilweises oder vollständiges Verdampfen des im Reaktionsgemisch enthaltenen Lösungsmittels zusammenführt.

3. Verfahren nach Anspruch 1, dadurch gekennzeichnet, daß man das Pseudojonon mit Methylenchlorid als Lösungsmittel und der Schwefelsäure unter intensiver Durchmischung und Siedekühlung durch teilweises oder vollständiges Verdampfen des im Reaktionsgemisch enthaltenen Methylenchlorids zusammenführt.

4. Verfahren nach Anspruch 1, dadurch gekennzeichnet, daß man das Pseudojonon mit dem Lösungsmittel und der Schwefelsäure unter intensiver Durchmischung und Siedekühlung so zusammenführt, daß die Temperatur des Reaktionsgemisches zwischen 35 und 45 °C liegt.

5. Verfahren nach Anspruch 1, dadurch gekennzeichnet, daß man auch das Verdünnen des Reaktionsgemisches mit Wasser unter Siedekühlung durch Verdampfen des im Reaktionsgemisch enthaltenen Lösungsmittels vornimmt.

6. Verfahren nach Anspruch 1, dadurch gekennzeichnet, daß man zur Herstellung von überwiegend α-Jonon das Pseudojonon mit dem Lösungsmittel und nur 2 bis 3 mol Schwefelsäure pro Mol Pseudojonon unter intensiver Durchmischung und Siedekühlung zusammenführt.

7. Verfahren nach Anspruch 1, dadurch gekennzeichnet, daß man zur Herstellung von überwiegend β-Jonon das Pseudojonon mit dem Lösungsmittel und mehr als 5 mol Schwefelsäure pro Mol Pseudojonon unter intensiver Durchmischung und Siedekühlung zusammenführt.

8. Verfahren nach den Ansprüchen 1 bis 7, dadurch gekennzeichnet, daß das Wasser, welches zur Verdünnung des Reaktionsgemisches verwendet wird, etwa 20-50 Gew.% Schwefelsäure enthält.

**Claims**

1. A process for the continuous preparation of ionones by cyclization of pseudoionone with concentrated sulfuric acid in the presence of an organic solvent or diluent, while cooling, and by dilution of the reaction mixture with water, wherein the pseudoionone is combined with an aliphatic or cycloaliphatic hydrocarbon which boils at 25-65 °C under the reaction conditions, or with an aliphatic chlorohydrocarbon, and the sulfuric acid, with intensive mixing and evaporative cooling by partial or complete vaporization of the solvent present in the reaction mixture, in such a way that the temperature of the reaction mixture is from 30 to 55 °C and the residence time of this mixture before it is diluted with water is from 0,05 to 20, preferably from 0,1 to 5, seconds.

2. A process as claimed in claim 1, wherein the pseudoionone is combined with a solvent which boils at 30-55 °C under atmospheric pressure and with the sulfuric acid, with intensive mixing and evaporative cooling by partial or complete vaporization of the solvent present in the reaction mixture.

3. A process as claimed in claim 1, wherein the pseudoionone is combined with methylene chloride as solvent and with sulfuric acid, with intensive mixing and evaporative cooling by partial or complete vaporization of the methylene chloride present in the reaction mixture.

4. A process as claimed in claim 1, wherein the pseudoionone is combined with the solvent and the sulfuric acid, with intensive mixing and evaporative cooling, in such a way that the temperature of the reaction mixture is from 35 to 45 °C.

5. A process as claimed in claim 1, wherein dilution of the reaction mixture with water is also carried out with evaporative cooling by vaporization of the solvent present in the reaction mixture.

6. A process as claimed in claim 1, wherein, in order to prepare predominantly α-ionone, the pseudoionone is combined with the solvent and only from 2 to 3 moles of sulfuric acid per mole of pseudoionone, with intensive mixing and evaporative cooling.

7. A process as claimed in claim 1, wherein, in order to prepare predominantly β-ionone, the pseudoionone is combined with the solvent and more than 5 moles of sulfuric acid per mole of pseudoionone, with intensive mixing and evaporative cooling.

8. A process as claimed in claims 1 to 7, wherein the water used for diluting the reaction mixture contrains about 20-50 % by weight of sulfuric acid.

**0 133 668**

### Revendications

1. Procédé pour la préparation en continu d'ionones par cyclisation de pseudo-ionone au moyen d'acide sulfurique concentré, en présence de solvants ou diluants organiques, sous refroidissement, et par dilution du mélange réactionnel avec de l'eau, caractérisé en ce qu'on fait arriver simultanément la pseudo-ionone avec un hydrocarbure aliphatique ou cycloaliphatique bouillant entre 25 et 65 °C dans les conditions de la réaction ou avec un hydrocarbure aliphatique chloré et l'acide sulfurique dans des conditions de mélange énergique et de refroidissement par vaporisation partielle ou complète du solvant contenu dans le mélange réactionnel, de telle manière que la température du mélange réactionnel se situe entre 30 et 55 °C et que le temps de séjour du mélange réactionnel jusqu'à la dilution à l'eau soit compris entre 0,05 et 20, de préférence entre 0,1 et 5 s.

2. Procédé selon la revendication 1, caractérisé en ce qu'on fait arriver simultanément la pseudo-ionone avec un solvant bouillant entre 30 et 55 °C sous la pression atmosphérique normale et l'acide sulfurique dans des conditions de mélange énergique et de refroidissement par vaporisation partielle ou complète du solvant contenu dans le mélange réactionnel.

3. Procédé selon la revendication 1, caractérisé en ce qu'on fait arriver simultanément la pseudo-ionone avec du chlorure de méthylène comme solvant et l'acide sulfurique dans des conditions de mélange énergique et de refroidissement par vaporisation partielle ou complète du chlorure de méthylène contenu dans le mélange réactionnel.

4. Procédé selon la revendication 1, caractérisé en ce qu'on fait arriver simultanément la pseudo-ionone avec le solvant et l'acide sulfurique dans des conditions de mélange énergique et de refroidissement par vaporisation, de telle manière que la température du mélange réactionnel se situe entre 35 et 45 °C.

5. Procédé selon la revendication 1, caractérisé en ce que la dilution du mélange réactionnel avec de l'eau est aussi effectuée sous refroidissement par vaporisation du solvant contenu dans le mélange réactionnel.

6. Procédé selon la revendication 1, caractérisé en ce que pour la préparation d'α-ionone, d'une manière prédominante, on fait arriver simultanément la pseudo-ionone avec le solvant et seulement 2 à 3 mol d'acide sulfurique par mol de pseudo-ionone, dans des conditions de mélange énergique et de refroidissement par vaporisation.

7. Procédé selon la revendication 1, caractérisé en ce que pour la préparation de β-ionone d'une manière prépondérante, on fait arriver simultanément la pseudo-ionone avec le solvant et plus de 5 mol d'acide sulfurique par mol de pseudo-ionone, dans des conditions de mélange énergique et de refroidissement par vaporisation.

8. Procédé selon l'une quelconque des revendications 1 à 7, caractérisé en ce que l'eau qui est utilisée pour la dilution du mélange réactionnel contient environ 20 à 50 % en poids d'acide sulfurique.